# EUROPÄISCHE PATENTSCHRIFT

(11) **EP 1 471 899 B1**
(45) Veröffentlichungstag und Bekanntmachung des Hinweises auf die Patenterteilung: **24.10.2007**
(21) Anmeldenummer: 03731681.7
(22) Anmeldetag: 21.01.2003
(51) Int. Cl.: A61K 31/136, A61P 25/06, A61P 25/02

(54) **VERWENDUNG VON AMBROXOL ZUR BEHANDLUNG VON CHRONISCHEN SCHMERZEN**
USE OF AMBROXOL IN THE TREATMENT OF CHRONIC PAIN
UTILISATION D'AMBROXOL DANS LE TRAITEMENT DE DOULEURS CHRONIQUES

(30) Priorität: 25.01.2002 DE 10203104
(43) Veröffentlichungstag der Anmeldung: 03.11.2004
(73) Patentinhaber: Boehringer Ingelheim Pharma GmbH & Co.KG, 55216 Ingelheim am Rhein (DE)
(72) Erfinder: GAIDA, Wolfram, 55218 Ingelheim (DE); KLINDER, Klaus, 88422 Oggelshausen (DE); WEISER, Thomas, 65189 Wiesbaden (DE)
(86) Internationale Anmeldenummer: PCT/EP2003/000531
(87) Internationale Veröffentlichungsnummer: WO 2003/061642

(56) Entgegenhaltungen:
- EP-A- 0 896 815
- WO-A-94/14476
- DATABASE BIOSIS [Online] BIOSCIENCES INFORMATION SERVICE, PHILADELPHIA, PA, US; 2000 WEISER T ET AL: "The secretolytic ambroxol is a potent inhibitor of neuronal sodium channels." Database accession no. PREV200000353898 XP009010115 & EUROPEAN JOURNAL OF NEUROSCIENCE, Bd. 12, Nr. Supplement 11, 2000, Seite 25 Meeting of the Federation of European Neuroscience Societies;Brighton, UK; June 24-28, 2000 ISSN: 0953-816X
- PATENT ABSTRACTS OF JAPAN vol. 2000, no. 23, 10. Februar 2001 (2001-02-10) & JP 2001 151677 A (TAISHO PHARMACEUT CO LTD), 5. Juni 2001 (2001-06-05)
- PATENT ABSTRACTS OF JAPAN vol. 2000, no. 06, 22. September 2000 (2000-09-22) & JP 2000 080034 A (TAISHO PHARMACEUT CO LTD), 21. März 2000 (2000-03-21)
- DATABASE EMBASE [Online] ELSEVIER SCIENCE PUBLISHERS, AMSTERDAM, NL; 25. April 2002 (2002-04-25) "Ambroxol for the treatment of throat pain" Database accession no. EMB-2002160473 XP001147779 & DEUTSCHE APOTHEKER ZEITUNG 25 APR 2002 GERMANY, Bd. 142, Nr. 17, 25. April 2002 (2002-04-25), Seiten 45-47, ISSN: 0011-9857
- DATABASE MEDLINE [Online] US NATIONAL LIBRARY OF MEDICINE (NLM), BETHESDA, MD, US; 2002 SCHUTZ ALEXANDER ET AL: "Local anaesthetic properties of ambroxol hydrochloride lozenges in view of sore throat. Clinical proof of concept." Database accession no. NLM11963647 XP001147862 & ARZNEIMITTEL-FORSCHUNG. GERMANY 2002, Bd. 52, Nr. 3, 2002, Seiten 194-199, ISSN: 0004-4172
- DATABASE EMBASE [Online] ELSEVIER SCIENCE PUBLISHERS, AMSTERDAM, NL; 1. September 2002 (2002-09-01) WEISER T ET AL: "Inhibition of tetrodotoxin (TTX)-resistant and TTX-sensitive neuronal Nachannels by the secretolytic ambroxol" Database accession no. EMB-2003117918 XP009010033 & MOLECULAR PHARMACOLOGY 01 SEP 2002 UNITED STATES, Bd. 62, Nr. 3, 1. September 2002 (2002-09-01), Seiten 433-438, ISSN: 0026-895X

## Beschreibung

Die Erfindung betrifft die Verwendung von Ambroxol und dessen pharmakologisch verträglichen Salze zur Herstellung eines Arzneimittels zur Behandlung von Krankheiten, welche auf einer starken Aktivierung spannungsabhängiger Natriumkanäle beruhen, insbesondere zur Behandlung chronischer Schmerzen.

### Hintergrund der Erfindung

Der Wirkstoff Ambroxol (trans-4-(2-Amino-3,5-dibrombenzylamino)-cyclohexanol) ist ein bekanntes Antitussivum und Expectorant. Darüberhinaus ist die Wirkung von Ambroxol als Natriumkanalblocker in der Literatur (Society for Neuroscience Abstracts, 2000, Vol.26, No. 1-2) beschrieben.

Die potentielle Wirkung von Natriumkanalblockern als Schmerzmittel ist ebenfalls aus dem Stand der Technik bekannt (Mao und Chen(2000), Pain 87, 7-17). Bekannte Natriumkanalblocker eignen sich jedoch nicht grundsätzlich zur Behandlung chronischer Schmerzen und Behandlung von Erkrankungen, deren Ursache in einer übermäßig starken Aktivierung von spannungsabhängigen Natriumkanälen liegt, da sie präferrentiell solche Natriumkanäle hemmen, die eine untergeordnete Rolle für die Entstehung und Weiterleitung noxischer Signale in sensorischen Neuronen spielen, d.h. solche, die durch Tetrodotoxin gehemmt werden können, im Gegensatz zu Tetrodotoxin-resistenten neuronalen Natriumkanälen. (Rush und Elliott (1997), Neuroscience Letters 226, 95-98; Scholz et al. (1998); Journal of Neurophysiology 79, 1746-1754;Song et al. (1997), Journal of Pharmacology and Experimental Therapeutics, 282, 707-714).

Zu Erkrankungen, die mit chronischem bzw. chronisch wiederkehrendem Schmerz verbunden sind, gehören u.a. Migräne, Neuralgien, Muskelschmerzen und Entzündungsschmerzen. Sie weisen mit dem chronisch wiederkehrenden Schmerz gemeinsame Mechanismen auf [Dray, A. Urban L. and Dickenson, A. Trends in Pharmacological Sciences 1994; 15:190-197].

Zu den chronisch neuronalen Schmerzen gehören u.a. postoperative Schmerzen, Gürtelrose, Phantomschmerz, diabetische Neuropathie, Schmerz nach chronischer Nervenkompression sowie Aids und Krebs im Finalstadium.

Es ist die Aufgabe der vorliegenden Erfindung einen Wirkstoff für die Behandlung von Erkrankungen, welche durch übermäßig starke Aktivierung von spannungsabhängigen Natriumkanälen bedingt sind, bereitzustellen.

Insbesondere liegt die Aufgabe der vorliegenden Erfindung in der Bereitstellung eines Wirkstoffs für die Behandlung chronischer Schmerzen, besonders chronisch neuronaler oder neuropathischer Schmerzen, mit guter Bioverfügbarkeit und starker antinocizeptiver Wirkung.

### Beschreibung der Erfindung

Überraschender Weise zeigt Ambroxol eine sehr gute Wirkung bei der Behandlung von chronischen Schmerzen und neurologischen Erkrankungen, die auf einer Blockade übermäßig stark aktivierter spannungsabhängiger Natriumkanäle, insbesondere übermäßig stark aktivierter spannnungsabhängiger neuronaler Natriumkanäle, beruht.

Die Erfindung betrifft daher die Verwendung von Ambroxol oder eines seiner pharmakologisch verträglichen Salze für die Herstellung eines Arzneimittels zur Behandlung von Krankheiten, welche auf einer starken Aktivierung spannungsabhängiger Natriumkanäle beruhen.

Bevorzugt ist die Verwendung von Ambroxol oder eines seiner pharmakologisch verträglichen Salze für die Herstellung eines Arzneimittels zur Behandlung von chronischen und/oder neuropathischen Schmerzen, vorzugsweise bei diabetischer Neuropathie, postherpatischer Neuralgie, chronischen Rückenschmerzen, Migräne, Trigeminusneuralgie oder Tumorschmerz, besonders bevorzugt bei diabetischer Neuropathie, postherpatischer Neuralgie, chronischen Rückenschmerzen oder Migräne, insbesondere bevorzugt bei diabetischer Neuropathie oder postherpatischer Neuralgie.

Ebenfalls bevorzugt ist die Verwendung von Ambroxol zur Behandlung von Schmerzpatienten oder Patienten mit Tumorerkrankungen.

Zur Salzbildung von Ambroxol geeignete Säuren sind beispielsweise Salzsäure, Bromwasserstoffsäure, Schwefelsäure, Phosphorsäure, Salpetersäure, Oxalsäure, Malonsäure, Fumarsäure, Maleinsäure, Weinsäure, Zitronensäure, Ascorbinsäure und Methansulfonsäure, vorzugsweise Salzsäure.

Die erfindungsgemäße Wirkung von Ambroxol soll durch nachfolgende Beispiele erläutert werden. Diese dienen lediglich zur Veranschaulichung der Erfindung und sind nicht als limitierend anzusehen.

Ambroxol zeigt eine antinocizeptive Wirkung, die auf einer Blockade spannungsabhängiger Natriumkanäle beruht. Im Gegensatz zu beschriebenen klinisch verwendeten Natriumkanalblockern inhibiert Ambroxol präferrentiell Tetrodotoxin-resistente Natriumkanäle in nocizeptiven C-Faser Neuronen. Deren besondere Relevanz für entzündlichen und chronische Schmerzzustände wurde in vivo belegt (Waxman et al. (1999) Proceedings of the National Academy of Science 96, 7635-7639; Khasar et al. (1998), Neuroscience Letters 256, 17-20).

In Neuronenkulturen aus den Hinterwurzelganglien adulter Ratten wurden Tetrodotoxin-resistente Natriumkanäle durch 35 µM Ambroxol halbmaximal gehemmt. Tetrodotoxin-sensitive Ströme wurden durch diese Konzentration weit schwächer inhibiert, hier lag die IC₅₀ höher als 100 µM.

Der tierexperimentelle Nachweis der stark analgetischen Wirkung der Kanalblockade wurde u.a. mittels des Formalin-Pfoten Tests an Ratten geführt. Der Test wird im folgenden beschrieben.

Formalin-Pfoten-Test (Ref: Carlton S M and Zhou S: Attenuation of formalin-induced nociceptive behaviors following local peripheral injection of gabapeptin. Pain 76, 201-207,1998).

Männliche Ratten (Chbb: THOM) im Gewicht von 250-300 Gramm werden benutzt. 20 µl einer 2% igen Formaldehyd-Lösung wird in die Plantarregion der rechten Hinterpfote injiziert. Unmittelbar danach werden eine Stunde lang die Anzahl der Flinches (Zuckungen der betroffenen Hinterpfote)-und die Dauer des Leckens der betroffenen Pfote registriert. Nach jeweils 5 Minuten werden die Werte zu Epochen zusammengefaßt. Aus den Epochenwerten werden Zeit-Wirkungs-Kurven für Flinches und Lecken erstellt. Typischerweise werden zwei Phasen der Formalin-Wirkung (Flinches, Lecken) beobachtet. Eine erste Phase von 0-10 Minuten und eine zweite Phase von 10-60 Minuten. Zwischen den beiden Phasen sinken Anzahl der Flinches und Dauer des Leckens gegen 0 (Interphase). Aus den Zeit-Wirkungs-Kurven werden die Flächen unter der Kurve für die erste Phase und für die zweite Phase ermittelt. Pro Kontrolle, Placebo und Substanzdosis werden üblicherweise 5 Tiere eingesetzt. Die Ergebnisse der Substanzdosen werden mit denen der Kontrolle verglichen und es werden ED₅₀-Werte kalkuliert. ED₅₀ ist die Dosis, bei der die Kontrollwerte um 50% gehemmt sind.

Der ED₅₀-Wert für Ambroxol Hydrochlorid beträgt 70 mg/kg p.o.

In weiteren Testmodellen für neuropathischen Schmerz in der Ratte ^{(1),(2)} reduzierte Ambroxol die taktile Allodynie, sowie die thermale Hyperalgesie.
⁽¹⁾ Bennett GJ and Xie Y-K. A peripheral mononeuropathy in rat that produces disorders of pain sensation like those seen in man. Pain 33, 87-107, 19883.
⁽²⁾ Seltzer, Z, Dubner R. and Shir, Y. A novel behavioral model of neuropathic pain disorder. Pain 43, 205-218, 1990.

Ambroxol kann allein oder in Kombination mit weiteren pharmakologisch aktiven Wirkstoffen zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbessemdes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z. B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt 30 und 4000 mg, bevorzugt 100 bis 2000 mg pro Erwachsenem.

Die nachfolgenden Beispiele illustrieren die vorliegende Erfindung ohne sie jedoch in ihrem Umfang zu beschränken:

### Pharmazeutische Formulierungsbeispiele

| A) | Tabletten | pro Tablette | |
|---|---|---|---|
| | Ambroxol | 800 | mg |
| | Milchzucker | 140 | mg |
| | Maisstärke | 240 | mg |
| | Polyvinylpyrrolidon | 20 | mg |
| | Magnesiumstearat | 10 | mg |

Ambroxol, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette | |
|---|---|---|---|
| | Ambroxol | 800 | mg |
| | Maisstärke | 190 | mg |
| | Milchzucker | 55 | mg |
| | Mikrokristalline Cellulose | 35 | mg |
| | Polyvinylpyrrolidon | 20 | mg |
| | Natrium-carboxymethylstärke | 30 | mg |
| | Magnesiumstearat | 10 | mg |

Ambroxol, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

| C) | Dragées | pro Dragée | |
|---|---|---|---|
| | Ambroxol | 500 | mg |
| | Maisstärke | 45 | mg |
| | Milchzucker | 30 | mg |
| | Polyvinylpyrrolidon | 5 | mg |
| | Magnesiumstearat | 5 | mg |

Ambroxol, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 11 mm gepreßt. Die so hergestellten Dragéekerne werden auf bekannte Weise mit einer Schicht überzogen, die im wesentlichten aus Zucker und Talkum besteht. Die fertigen Dragées werden mit Wachs poliert.

| D) | Kapseln | pro Kapsel | |
|---|---|---|---|
| | Ambroxol | 250 | mg |
| | Maisstärke | 268,5 | mg |
| | Magnesiumstearat | 1,5 | mg |

Ambroxol und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

| E) | Parenterale Lösung | | |
|---|---|---|---|
| | Ambroxol | 500 | mg |
| | Citronensäure-Monohydrat | 100 | mg |
| | Natriumhydroxid | 35 | mg |
| | Mannit | 1500 | mg |
| | Aqua pro inj. | 50 | ml |

Ambroxol wird bei Eigen-pH oder gegebenenfalls bei pH 4,5 bis 5,5 in Wasser gelöst und mit Mannit als Isotonans versetzt. Die erhaltene Lösung wird pyrogenfrei filtriert und das Filtrat unter aseptischen Bedingungen in Injektionsflaschen abgefüllt, die anschließend mit Gummistopfen verschlossen und autoklaviert werden.

| F) | Suppositorien | | |
|---|---|---|---|
| | Ambroxol | 450 | mg |
| | Adeps solidus | 1650 | mg |

Das Hartfett wird geschmolzen. Bei 40°C wird Ambroxol homogen dispergiert. Es wird auf 38°C abgekühlt und in schwach vorgekühlte Suppositorienformen ausgegossen.

| G) | orale Lösung | | |
|---|---|---|---|
| | Ambroxol | 150 | mg |
| | Hydroxyethylcellulose | 50 | mg |
| | Sorbinsäure | 5 | mg |
| | Sorbit (70%ig) | 600 | mg |
| | Glycerin | 200 | mg |
| | Aroma | 15 | mg |
| | Wasser ad | 10 | ml |

Destilliertes Wasser wird auf 70°C erhitzt. Hierin wird unter Rühren Hydroxyethylcellulose gelöst. Nach Zugabe von Sorbitlösung und Glycerin wird auf Raumtemperatur abgekühlt. Bei Raumtemperatur werden Sorbinsäure, Aroma und Ambroxol zugegeben. Zur Entlüftung der Suspension wird unter Rühren evakuiert.

| H) | Salbe | | |
|---|---|---|---|
| | Zusammensetzung g/100 g Salbe | | |
| | Ambroxol | 20 | g |
| | Natriumdisulfit | 0,1 | g |
| | Cetylalkohol | 10 | g |
| | Stearylalkohol | 10 | g |
| | Weiße Vaseline | 5 | g |
| | Parfümöl q.s. | | |
| | Destilliertes Wasser ad | 100 | g |

Die Bestandteile werden in üblicher Weise zu einer Salbe verarbeitet.

## Patentansprüche

1. Verwendung von Ambroxol oder eines seiner pharmakologisch verträglichen Salze für die Herstellung eines Arzneimittels zur Behandlung von chronischen Schmerzen.

2. Verwendung von Ambroxol nach Anspruch 1 zur Behandlung von Schmerzpatienten oder Patienten mit Tumorerkrankungen.

## Claims

1. Use of ambroxol or one of the pharmacologically acceptable salts thereof for preparing a pharmaceutical composition for the treatment of chronic pain.

2. Use of ambroxol according to claim 1 for the treatment of patients suffering pain or patients with tumoral diseases.

## Revendications

1. Utilisation de l'ambroxol ou de l'un de ses sels pharmacologiquement acceptables pour la production d'un médicament pour le traitement des douleurs chroniques.

2. Utilisation de l'ambroxol selon la revendication 1 pour le traitement des patients ressentant des douleurs ou des patients ayant des maladies tumorales.
